# EUROPEAN PATENT APPLICATION

(11) **EP 4 696 307 A1**
(43) Date of publication of application: **18.02.2026**
(21) Application number: 25196020.9
(22) Date of filing: 14.08.2025
(51) Int. Cl.: A61K 31/225, A61K 31/015, A61K 31/045, A61K 31/10, A61K 45/06, A61P 3/10, A61P 7/00, A61P 7/06, A61P 29/00

(54) **COMPOSITIONS AND METHODS FOR ENHANCING THE EFFICACY OF DICARBOXYLIC ACID ESTERS, AND USES THEREOF**

(30) Priority: 15.08.2024 US 202418806476
(71) Applicant: New Frontier Labs, LLC, San Antonio, TX 78240 (US)
(72) Inventor: IZBICKA, Elzbieta, San Antonio, Texas 78240 (US); STREEPER, Robert T., San Antonio, Texas 78240 (US)
(74) Representative: Vossius & Partner Patentanwälte Rechtsanwälte mbB

(57) **Abstract**

This disclosure provides methods and compositions including dicarboxylic acid esters, and at least one solvent that is effective in enhancing the therapeutic efficacy of the dicarboxylic acid ester. In some embodiments, the composition further includes at least one terpene, and other embodiments include methods of making the compositions and uses of the compositions for treating a variety of ailments that dicarboxylic acid esters are useful in treating.

## Description

### FIELD OF THE EMBODIMENTS

This disclosure relates to methods and pharmaceutical compositions including dicarboxylic acid esters, and at least one solvent that are effective in enhancing the therapeutic efficacy of the dicarboxylic acid ester. In some embodiments, the composition further includes at least one terpene, and other embodiments include methods of making the compositions and uses of the compositions for treating a variety of ailments that dicarboxylic acid esters are useful in treating.

### BACKGROUND

Diethyl azelate (DEA) exhibits unexpectedly diverse useful medical activities in oral and topical administration for the management of insulin resistance, muscoskeletal pain and envenomation with phospholipases present in animal venoms. *See, e.g.,* U.S. Patent Nos. 10,251,857 B2, 11,026,912 B2, 11,918,555, and 11,911,358, the disclosures of which are incorporated herein by reference in their entireties. See *also,* Streeper RT and Izbicka E, "Diethyl azelate for the treatment of brown recluse spider bite, a neglected orphan indication," In Vivo 36(1): 86-93, (2022).

Anesthetic and analgesic drugs are widely utilized for the treatment of pain. Recently, Pavel *et al.* describe in detail the biochemical mechanisms by which general anesthetics exert their pharmacological effects. Pavel shows that inhalational anesthetics disrupt the ordering of lipid rafts and by dint of this disruption induce anesthesia. The best-studied lipid rafts are membrane domains enriched in cholesterol and sphingomyelin (e.g., monosialotetrahexosylganglioside1 aka GM1) that bind cholera toxin B subunit (CTxB). GM1 lipid rafts contain phospholipase D2 (PLD2). Inhalational anesthetics increase the fluidity of, and thereby disrupt, GM1 lipid rafts causing PLD2 to depart the lipid raft and translocate to the proximity of TREK1. Once there, PLD2 hydrolyzes phosphatidylcholine (PC) to produce phosphatidic acid (PA) and choline. The PA then binds to and activates TREK1. When TREK1 is activated the resultant potassium influx induces unconsciousness and analgesia. Pavel *et al.* also show that PLD2 translocation also activates a second type of channel called TRAAK which is an anesthetic-insensitive homolog of TREK-1. [Pavel MA, et al., PNAS, (2020) 117(24):13757-66].

The inventors have discovered that dicarboxylic acid esters of the present disclosure, when combined with at least one solvent and at least one terpene are surprisingly effective for the treatment of a variety of disorders, including pain, diabetes, and exposure to brown recluse spider toxins and bee venom. The inventors have discovered that when combined with at least one solvent and at least one terpene, the dicarboxylic acid ester produces beneficial synergistic effects.

Dimethyl sulfoxide (DMSO)is an organosulfur compound that enhances the plasma membrane permeation of drugs, DNA and other substances. Increasing concentrations of DMSO reportedly induce thinning of the plasma membrane followed by pore formation and, finally, a collapse of the bilayer. [de Menorval MA, et al., "Effects of dimethyl sulfoxide in cholesterol-containing lipid membranes: A comparative study of experiments in silico and with cells," PLoS One Vol 7(7): e41733, (2012)]. DMSO induces rapid, modest and short-lived relief of arthritic pain [Swanson B, "Medical use of dimethyl sulfoxide (DMSO)," Rev Clin Basic Pharm, Vol. 5, pp. 1-33, (1985)], and has some utility in wound healing; Capriotti K, et al., "Dimethyl sulfoxide: History, chemistry, and clinical utility in dermatology," J Clin Aesthet Dermatol, Vol. 5(9): 24-26, (2012)]. DMSO applied to the skin quickly causes a distinctive garlic taste on the tongue that is indicative of its rapid transport through tissues and around the body. [Horita A, et al., "Skin penetrating property of drugs dissolved in dimethyl sulfoxide (DMSO) and other vehicles," Live Sciences, Vol. 3, pp.1389-1395, (1964)]. The oral use of DMSO has been limited to the treatment of systemic amyloid A amyloidosis, a complication of chronic inflammatory disease.

Terpenes also enhance skin penetration by interaction with intercellular lipids in stratum corneum, the outermost layer of the skin. Terpenes can boost the activity of both hydrophilic and lipophilic drugs even at low concentrations. [Chen J, et al., "Natural terpenes as penetration enhancers for transdermal drug delivery," Molecules, vol. 21, pg. 1709, (2016)]. Representative terpenes such as limonene and menthol exhibit low cytotoxicity *in vivo* and improve bioavailability of drugs in animal models and in human skin. Limonene has anti-inflammatory activity when applied to the skin and facilitates wound healing *in vivo.* [d'Alessio PA, et al., "Skin repair properties of d-limonene and perillyl alcohol in murine models," Antiinflamm Antiallergy Agents Med Chem, Vol 13(1): pp. 29-35, (2014)]. Menthol can stimulate skin nociceptors, initiate release of vasodilator peptides and increase skin temperature.

At the most fundamental level, all types of pain, other than psychogenic pain, involve the transmission of aversive or noxious stimuli to the central nervous system via the afferent nerves. The nerve impulse transmission that occurs must at multiple points in the chain of signal transmission cross cellular plasma membranes [Basbaum A, et al., Cell. (2009)139(2):267-84]. The role of the plasma membrane in the mechanism of pain has been demonstrated *in vivo* whereby disruption of nerve signaling by increasing lipid raft fluidity via depletion of cholesterol by cyclodextrin reversibly diminished hyperalgesia induced by prostaglandin E2 (PGE2) [Ferrari, L, et al., J. Pain (2015)16(1):60-6].

Most pain conditions in humans and in animal pain models result in marked alterations in mechanical sensation whereby the mechanical force initially affects the plasma membrane. More light on the process has been shed by a finding of the link between mechanical stimulus and signaling from membrane-associated phospholipase D2 (PLD2), a mechanosensitive enzyme that resides in a membrane-lipid site comprised of cholesterol, ganglioside GM1 and the mechanically activated ion channel TREK-1 that is responsible for downstream signaling. [Petersen EN, et al., "Mechanical activation of TWIK-related potassium channel by nanoscopic movement and rapid second messenger signaling," Elife, Vol. 12 (2024)]. A common experimental method to gauge the level of stimulus-evoked skin sensitivity employs a set of graded monofilaments known as von Frey fibers. [Mills C, et al., "Estimating efficacy and drug ed50's using von Frey thresholds: Impact of Weber's law and log transformation," J Pain, Vol. 13(6), pp. 519-523, (2012)].

Other members of the phospholipase family represented by phospholipase A2 (PLA2) are expressed in diverse species ranging from bacteria to humans. PLA2s induce pain and inflammation and in some cases exhibit hemolytic activity. Inhibition of PLA2 is viewed as a therapeutic target. [Yedgar S, et al., "Inhibition of phospholipase A(2) as a therapeutic target," Biochim Biophys Acta, Vol. 1488(1-2), pp. 182-187, (2000)]. DEA and related azelates have been reported to inhibit PLA2 enzymatic and hemolytic activity of bee and snake venoms. [Streeper RT and Izbicka E, "Diethyl azelate for the treatment of brown recluse spider bite, a neglected orphan indication," In Vivo 36(1): 86-93, (2022)].

The inventors have also demonstrated that dicarboxylic acid esters induce a number of changes in cellular and organismal signaling in ways that are therapeutically useful. In addition to their other pharmacological activities, dicarboxylic acid esters induce the cellular release and/or production of signaling molecules that have analgesic properties. Responsive cellular molecules include leptin, macrophage colony stimulating factor (M-CSF), granulocyte colony stimulating factor (G-CSF), and granulocyte-macrophage colony stimulating factor (GM-CSF). In addition, the inventors have discovered that treatment of mammalian cells and tissues with dicarboxylic acid esters can suppress the production of markers associated with pain, which include inducible nitric oxide synthase (iNOS) [Kwok Y, et al., PLZhang J, et al., Int. Anesthesiol. Clin. (2007) 45(2):27-37], prostaglandin E2 (PGE2) [ Ahlawat A, et al., Eur. J. Pharmacol. (2018) 818:419-28], and adenosine triphosphate (ATP) [Mense S, Dtsch Arztebl Int 2008; 105(12): 214-9].

There is a need in the art to enhance the efficacy of dicarboxylic acid esters, including diethyl azelate, in treating pain, decreasing blood glucose levels, inhibiting hemolysis and/or associated pain induced by PLA2, PLD and other phospholipases exemplified by PLA2, and other conditions and disorders. The embodiments described herein address and satisfy these needs and other needs readily apparent to those skilled in the art upon review of the embodiments described hereinafter.

### SUMMARY

Aspects of the present disclosure provides methods and pharmaceutical compositions including a dicarboxylic acid ester of Formula I:

R₂OOC-(CH₂)ₙ-COOR₁

wherein n is between 4 and 10, and each R₁ and R₂ are independently a lower alkyl, at least one solvent, and at least one terpene. Certain embodiments herein relate to inducing an analgesic effect in a subject, thereby inhibiting pain due in part to the activation of pattern recognition receptors (PRR) including Toll-like receptors (TLRs), nucleotide oligomerization domain (NOD) receptors, and/or Dectin receptors.

Certain embodiments herein relate to inducing an analgesic effect, thereby inhibiting a localized pain in a subject, by promoting the secretion of leptin, macrophage colony-stimulating factor (M-CSF), granulocyte macrophage colony-stimulating factor (GM-CSF), and/or granulocyte colony-stimulating factor (G-CSF).

In some aspects, embodiments herein relate to methods and pharmaceutical compositions including a dicarboxylic acid ester of Formula I, at least one solvent, and at least one terpene for inducing an analgesic effect, thereby inhibiting a localized pain in a subject, by suppressing the expression and secretion of prostaglandin E2 (PGE2) and/or inducible nitric oxide synthase (iNOS) and/or adenosine triphosphate (ATP).

In some aspects, embodiments herein relate to methods of inducing an analgesic effect, thereby inhibiting localized pain in a subject, the method including the step of administering to the subject in need a composition comprising a dicarboxylic acid ester of Formula I, at least one solvent, and at least one terpene, wherein administration results in a reduction in a symptom associated with the pain condition.

In some aspects, embodiments herein relate to methods of treating Type 2 diabetes, insulin resistance, and other disorders caused by high glucose levels by administering to the subject in need a composition comprising a dicarboxylic acid ester of Formula I, at least one solvent, and at least one terpene, wherein administration results in a reduction of blood glucose levels.

In some aspects, embodiments herein relate to methods of treating disorders, diseases and conditions associated with PLA2 and or PLD with pathological exogenous or endogenous phospholipase activities by administering to the subject in need a composition comprising a dicarboxylic acid ester of Formula I, at least one solvent, and at least one terpene, wherein administration results in an inhibition of hemolysis induced by PLA2 or PLD.

In some aspects, embodiments herein relate to uses of a dicarboxylic acid ester of Formula I, at least one solvent, and at least one terpene, in the manufacture of a medicament for inducing an analgesic effect, thereby inhibiting localized pain in a subject. In some aspects, embodiments herein relate to uses of a composition comprising a dicarboxylic acid ester of Formula I, at least one solvent, and at least one terpene, for reducing glucose levels, thereby treating Type 2 diabetes, insulin resistance and other disorders, diseases, or conditions caused by increased blood glucose levels.

In some aspects, embodiments herein relate to uses of a composition comprising a dicarboxylic acid ester of Formula I, at least one solvent, and at least one terpene, in the manufacture of a medicament for inhibiting hemolysis, including hemolysis induced by PLA2 and or PLD in a subject, thereby treating diseases and conditions caused by or associated with PLD and or PLA2.

### DESCRIPTION OF THE DRAWINGS

The following drawings demonstrate certain aspects of the present disclosure. The disclosure may be better understood by reference to one or more of these drawings in combination with the detailed description of specific embodiments presented herein.
**FIG. 1** show the variation of analgesic effect with increasing DMSO concentration in combination with DEA on the duration of the sensitivity response in cutaneous mechanical sensitivity assays.
**FIGS. 2A-2B** show the dose response of limonene in combination with DEA and DMSO assessed by the duration of sensitivity suppression in cutaneous mechanical sensitivity assays. **FIG. 2A** show the dose response of limonene with respect to the corrected time to baseline, and **FIG. 2B** show the dose response of limonene with respect to AUC for corrected time to baseline.

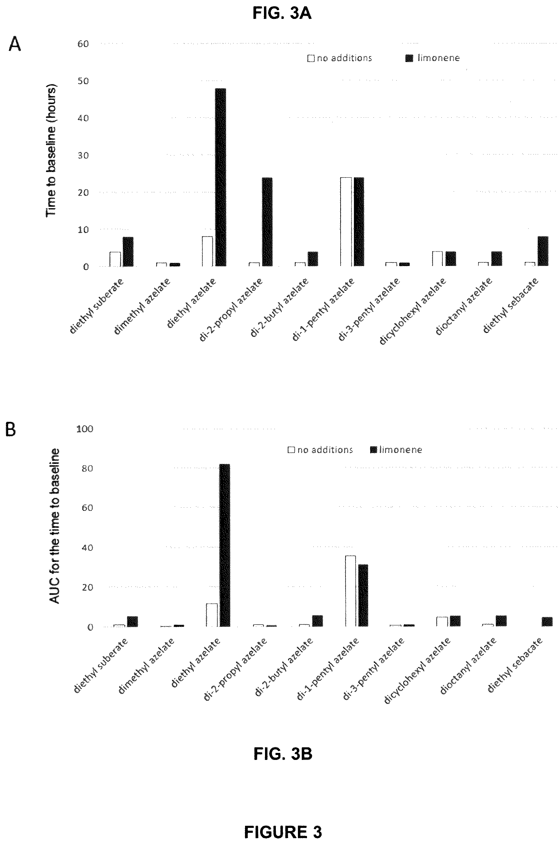

**FIGS. 3A-3B** show the effects of several related diesters of medium chain fatty acids and DMSO (0.5M each) alone and in combinations with 2% limonene on the duration of sensitivity suppression in cutaneous mechanical sensitivity assays. **FIG. 3A** show the effect of several related diesters of medium chain fatty acids and DMSO (0.5M each) alone and in combinations with 2% limonene on the time to baseline, and **FIG. 3B** show the effect of several related diesters of medium chain fatty acids and DMSO (0.5M each) alone and in combinations with 2% limonene on the AUC for the time to baseline.
**FIG. 4** show the effects of DEA, DMSO, limonene and menthol as single agents and in combinations thereof on the duration of sensitivity suppression in cutaneous mechanical sensitivity assays.
**FIG.** 5 demonstrates the superiority of the orally administered 77/21/2 mixture of DEA/DMSO/limonene versus DEA alone on blood glucose levels of a diabetic subject.
**FIG. 6** demonstrates the superiority of the 77/21/2 combination of DEA, DMSO and limonene versus individual agents in the inhibition of hemolysis induced by phospholipase A2.

### DETAILED DESCRIPTION

All applications, publications, patents and other references, cited herein are incorporated by reference in their entirety.

As used herein, the singular forms "a," "an" and "the" include plural referents unless the context clearly dictates otherwise. The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements.

Ranges may be expressed herein as from "about" one particular value, and/or to "about" another particular value. As used herein, the term "about," is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

As used herein, all numerical values or numerical ranges include integers within such ranges and fractions of the values or the integers within ranges unless the context clearly indicates otherwise. In addition, such ranges are also intended to include the numbers themselves and any sub-range between them. This range may be integral or continuous between and including the end values. Thus, for example, reference to a range of about 3 hours to about 10 hours, includes 3 hours, 4 hours, 5 hours, etc., as well as 3 hours and 1 minute, 3 hours and 2 minutes, 3 hours and 4 minutes, etc., 4 hours and 1 minute, 4 hours and 2 minutes, 4 hours and 4 minutes, etc. and so forth. Reference to a range of 90-100% includes 92.2% to 97.5%, 91.5 to 94.5, etc. Reference to a range of 1 to 25 days include sub-ranges from 1 day to 5 days, or from 3 days to 7 days, or from 5 days to 25 days.

As used herein, the term "comprising" is intended to mean that the pharmaceutical compositions (or compositions) and methods include the recited elements, but not excluding others. The term "consisting essentially of," as applied to the compositions of the present embodiments, means the composition can contain additional elements as long as the additional elements do not materially alter the composition. The term "materially altered," as applied to a composition, refers to an increase or decrease in the therapeutic effectiveness of the composition as compared to the effectiveness of a composition consisting of the recited elements. In other words, "consisting essentially of" when used to define compositions, shall mean excluding other components of any essential significance to the composition. Thus, a composition consisting essentially of the components as defined herein would not exclude trace contaminants from the isolation and purification method and pharmaceutically acceptable carriers. "Consisting of" shall mean excluding more than trace elements of other ingredients and substantial method steps for administering the compositions of this invention. Embodiments defined by each of these transition terms are within the scope of this invention.

As used herein, the term "optional" or "optionally" means that the subsequently described event or circumstance may or may not occur, and that the description includes instances where said event or circumstance occurs and instances where it does not.

As used herein, a "subject" refers to a mammal such as a primate, in one embodiment as a human. Non-human primates include marmosets, monkeys, chimpanzees, gorillas, orangutans, and gibbons, to name a few. The term "subject" includes cats, dogs, ferret, chinchilla, mouse, rabbit, rat, gerbil, guinea pig, cattle, horses, pigs, sheep, goats, chickens, turkeys, ducks, pheasants, pigeons, doves, parrots, cockatoos, geese, etc.

As used herein, a "subject in need" of the methods of the disclosure can be a subject suffering from a pain condition.

As used herein, the term "therapeutically effective" amount, refers to an amount of an active ingredient that is sufficient to induce a localized analgesic effect in a subject. The therapeutically effective amount of the pharmaceutical composition of the present disclosure may be effective in disrupting a lipid raft. The therapeutically effective amount of the pharmaceutical composition of the present disclosure may be effective in upregulating (e.g., stimulating) expression and/or secretion of leptin, M-CSF, G-CSF, GM-CSF etc. from a cell of the subject. The therapeutically effective amount of the pharmaceutical composition of the present disclosure may also be effective in downregulating (e.g., suppressing) expression and/or secretion of ATP, iNOS, PGE2, etc. from a cell of the subject. Alternatively stated, a "therapeutically effective" amount is an amount that will provide some alleviation, mitigation, decrease, or stabilization in at least one clinical symptom in the subject. Those skilled in the art will appreciate that the therapeutic effects need not be complete or curative, as long as some benefit is provided to the subject. The effective amount may vary depending on such factors as the wound or affected area, or the disease or condition being treated, the particular targeted constructs being administered, the size of the subject, or the severity of the disease or condition. One of ordinary skill in the art may empirically determine the effective amount of a particular composition without necessitating undue experimentation.

The term "active ingredient" used herein refers to a biologically active substance. In embodiments, the dicarboxylic acid ester of the present disclosure is the active ingredient in a pharmaceutical composition. In embodiments, the dicarboxylic acid ester of the present disclosure is the only active ingredient in a pharmaceutical composition. In embodiments, the azelaic acid ester of the present disclosure is the active ingredient in a pharmaceutical composition. In embodiments, diethyl azelate is the active ingredient in a pharmaceutical composition.

As used herein, the term "pharmaceutically acceptable carrier" refers to any suitable adjuvants, carriers, excipients, or stabilizers, and can be in solid or liquid form such as, tablets, capsules, powders, solutions, suspensions, or emulsions, that does not cause significant irritation to the subject and does not abrogate the biological activity and properties of the administered active ingredients.

As used herein, the term "disease" is intended to be generally synonymous, and is used interchangeably with the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

As used herein, the "treating" or "treatment" of a disease or condition, may refer to preventing the disease or condition, slowing the onset or rate of development of the disease or condition, reducing the risk of developing the disease or condition, preventing or delaying the development of symptoms associated with the disease or condition, reducing or ending symptoms associated with the disease or condition, generating a complete or partial regression of the disease or condition, or some combination thereof. Treatment may also mean a prophylactic or preventative treatment of a disease or condition.

As used herein, the term "inhibiting" refers to a decrease, reduction, limiting, and/or blocking of a particular action, function, interaction, appearance of a symptom. The terms "inhibiting," "decreasing," and "reducing" are used interchangeably herein. In embodiments, the term refers to reducing or preventing the level of certain activities, functions, interactions, appearances of certain symptoms (e.g., levels of biomarkers including, but not limited to, proteinaceous or non-proteinaceous molecules in tissues and body fluids) in a subject to a quantity which is at least 10%, 15%, 20%, 25%, 30%, 35%, 40%, 45%, 50%, 55%, 60%, 65%, 70%, 75%, 80%, 85%, 90%, 95%, 99% or less than the quantity in a corresponding control. For example, pain is decreased, reduced, or inhibited, if an indicator of pain, e.g., blood levels of prostaglandin PGE2, is at least about 10%, 20%, 30%, 50%, 80%, or 100% reduced. In embodiments, the pain is decreased, reduced, or inhibited by at least about 1-fold, 2-fold, 3-fold, 4-fold, or more in comparison to the pain prior to administration of the dicarboxylic acid ester of the present disclosure. The measurable change may be objective (*e.g*., measurable by some test or marker, for example, in an *in vitro* or in *vivo assay* or test or observation, or subjective (e.g., the subject gives an indication of or feels an effect).

As used herein, the term "pain" refers to an unpleasant sensory and emotional experience associated with actual or potential tissue damage, or described in terms of such damage and includes the more or less localized sensation of discomfort, distress, or agony, resulting from the stimulation of specialized nerve endings. There are many types of pain, including, but not limited to, nociceptive pain, neuropathic pain, or nociplastic pain, whether acute or chronic. The goal of inducing an analgesic effect in a subject is to reduce the degree or severity of pain perceived by the subject.

As used herein, the phrase "localized pain" refers to non-systemic pain experienced at a particular location or area on a specific part of the body of a subject. In embodiments, the area of pain is related to a primary lesion.

As used herein, the term "envenomation" refers to contact or injection of venom into a subject resulting from a bite, fang, tooth, or sting from a venomous carrier. The term "envenomation" also refers to injection from a venom delivery apparatus. As used herein, the term "venom" refers to any poisonous or toxic substance which is subcutaneously, intramuscularly or topically transmitted, by the bite or sting or in contact of a venomous animal into a subject, which may contain various toxins such as, but not limited to, hemotoxins, cytotoxins, myotoxins, neurotoxins. As used herein, the term "venomous carrier" refers to any animal or organism that produces, secrets, or carries a venom. Examples of venomous animals include, but are not limited to insect, reptile, amphibian, arthropod, mollusk, cnidarian, coelenterate or other venomous vertebrate or invertebrate animal. Examples of venomous organisms include but are not limited to bacteria or fungi.

As used herein, the term "dermonecrotic arachnidism" is intended to be generally synonymous, and is used interchangeably with the terms "necrotic arachnidism" or "necrosis" or "dermonecrosis" or "tissue necrosis," refers to the local skin and tissue injury as a result of envenomation or contact with PLD toxin.

As used herein, the term "skin ulcer" or "ulceration" refers to an open sore or wound on the skin wherein the epidermis is absent or otherwise compromised. The underlying dermis or hypodermis may be exposed and visible. The surrounding skin may be reddened and inflamed. The cardinal symptoms and signs of any kind of inflammatory process are pain, redness, heat, swelling, pain and loss of function. Such open sores may be prone to infection by pathogens such as bacteria, fungi, and viruses. In advanced cases, the sore may be oozing fluid-pus. Pus (dead immune cells, skin cells, subcutaneous tissue cells, cell fluid, and infectious agents) accumulate in the cavity of the skin ulcer to form an abscess.

"Diabetes" refers to a group of metabolic diseases characterized by high blood sugar (glucose) levels which result from defects in insulin secretion or action, or both. "Type 2 diabetes" or "T2D" refers to one of the two major types of diabetes, the type in which the beta cells of the pancreas produce insulin, at least in the early stages of the disease, but the body is unable to use it effectively because the cells of the body are resistant to the action of insulin. In later stages of the disease the beta cells may stop producing insulin. Type 2 diabetes is also known as insulin-resistant diabetes, non-insulin dependent diabetes and adult-onset diabetes.

"Pre-diabetes" refers to one or more early diabetes-related conditions including impaired glucose utilization, abnormal or impaired fasting glucose levels, impaired glucose tolerance, impaired insulin sensitivity and insulin resistance.

"Insulin resistant" refers to the condition when cells become resistant to the effects of insulin-a hormone that regulates the uptake of glucose into cells-or when the amount of insulin produced is insufficient to maintain a normal glucose level. Cells are diminished in the ability to respond to the action of insulin in promoting the transport of the sugar glucose from blood into muscles and other tissues (i.e. sensitivity to insulin decreases). Eventually, the pancreas produces far more insulin than normal and the cells continue to be resistant. As long as enough insulin is produced to overcome this resistance, blood glucose levels remain normal. Once the pancreas is no longer able to keep up, blood glucose starts to rise, resulting in diabetes. Insulin resistance ranges from normal (insulin sensitive) to insulin resistant (IR).

"Obesity" refers to a chronic condition defined by an excess amount body fat. The normal amount of body fat (expressed as percentage of body weight) is between 25-30% in women and 18-23% in men. Women with over 30% body fat and men with over 25% body fat are considered obese.

The vast literature of tissue penetration enhancers rarely directly addresses the enhancement of the efficacy of therapeutic molecules; usually the increases in drug tissue concentration is the sole focus, and it is typically assumed that increased concentration correlates with enhanced efficacy. The inventors unexpectedly discovered that that penetration and efficacy are NOT linearly correlated. For example, the efficacy of DEA in combination with a solvent such as DMSO follows a bell curve with a sharp peak at approximately 21% solvent and significantly lower efficacy below and above that concentration. If penetration were the sole driver of increased efficacy, such a marked peak would not be observed. While not intending on being bound by any theory of operation or function, the inventors believe that, for the DEA/solvent/terpene combination, efficacy is enhanced within a narrow and specific mole ratio of DEA to solvent and that the efficacy of a preferred ratio of DEA/solvent is further potentiated by a preferred quantity of at least one terpene, such as limonene and menthol. The embodiments herein show that penetration enhancement does not play a substantial role in enhanced efficacy of DEA.

### Pharmaceutical Compositions

In embodiments, the disclosure provides methods and pharmaceutical compositions for inducing an analgesic effect, thereby inhibiting pain in a subject, including administering to a subject a pharmaceutical composition including a dicarboxylic acid ester having the Formula I:

R₂OOC-(CH₂)ₙ-COOR,

at least one solvent, and at least one terpene. In embodiments, n is between 4 and 10, between 6 and 9, or between 7 and 8. In embodiments, each R₁ and R₂ are independently a lower alkyl. The term "lower alkyl," as used herein, refers to a C1 to C6 saturated straight or branched alkyl group. Example of suitable lower alkyl groups (R₁ and R₂) in Formula I include methyl, ethyl, propyl, isopropyl, butyl, sec-butyl, t-butyl, and the like groups. In embodiments, R₁ and R₂ are the same. In embodiments, R₁ and R₂ are different.

In embodiments, the dicarboxylic acid ester is an azelaic acid ester. In embodiments, the azelaic acid ester is diethyl azelate, diisopropyl azelate, or mixtures thereof. In embodiments, the azelaic acid ester is diethyl azelate.

In embodiments, the dicarboxylic acid ester of Formula I can be administered in an amount sufficient to induce an analgesic effect in a subject, such that to reduce a pain response. In embodiments, the dicarboxylic acid ester of Formula I may be administered in an amount sufficient to reduce a pain response by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In embodiments, the dicarboxylic acid ester of Formula I can be administered in an amount sufficient to reduce the blood glucose levels in a subject to treat one or more disorders, diseases, or conditions caused by enhanced blood glucose levels (e.g., insulin resistance, Type II diabetes). In another embodiment, the dicarboxylic acid ester of Formula I can be administered in an amount sufficient to inhibit hemolysis caused by a toxin such that the administration can treat disorders, diseases and conditions associated with PLD toxins.

In embodiments, the dicarboxylic acid ester of Formula I is administered in an amount sufficient to provide the above-described effects, and can be administered in a range from, e.g., about 10% to about 90%, about 20% to about 90%, about 30% to about 90%, about 40% to about 90%, about 50% to about 90%, about 60% to about 90%, about 70% to about 90%, about 70% to about 80%, or any value or range therebetween.

The solvent used with the active ingredient dicarboxylic acid ester, preferably diethyl azelate, can be any solvent capable of enhancing the efficacy of the active ingredient. Solvents may be selected from dimethyl formamide, trifluoroacetic acid, dimethyl sulfoxide (DMSO), dihydrolevoglucosenone (CYRENE^{™}), N-methylpyrollidone (NMP), halogenated analgesics selected from halothane, isoflurane, enflurane, deslufrane, and sevoflurane, and mixtures and combinations thereof. In an embodiment, the solvent is DMSO.

In embodiments, the solvent may be administered in an amount sufficient to enhance the therapeutic efficacy of the dicarboxylic acid ester of Formula I. In certain embodiments, the solvent, e.g., DMSO, may be administered in an amount sufficient to reduce a pain response by, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95%. In embodiments, the solvent, or DMSO, can be administered in an amount sufficient to enhance the therapeutic efficacy of the dicarboxylic acid ester of Formula I in reducing the blood glucose levels in a subject to treat one or more disorders, diseases, or conditions caused by enhanced blood glucose levels (e.g., insulin resistance, Type 2 diabetes). In another embodiment, the solvent, or DMSO, can be administered in an amount sufficient to enhance the therapeutic efficacy of dicarboxylic acid ester of Formula I in inhibiting hemolysis caused by a toxin such that the administration can treat disorders, diseases and conditions associated with PLD toxins.

In embodiments, the solvent can be administered in an amount sufficient to enhance the efficacy of the dicarboxylic acid ester in providing the effects described above. The one or more solvent may be used in an amount within the range from, e.g., about 1% to about 80%, about 5% to about 70%, about 10% to about 50%, about 15% to about 40%, about 20% to about 30%, or any value or range therebetween.

The at least one terpene or related terpene used with the active ingredient dicarboxylic acid ester, preferably diethyl azelate, can be any terpene capable of enhancing the efficacy of the active ingredient. The at least one terpene or related terpene may be a terpenoid, where the terpene or terpenoid has a mono-cyclic or bicyclic structure, and/or the terpene or terpenoid has ten carbon atoms in the chemical formula. The terpene or terpenoid may be selected from the group consisting of arnica extract, arnica oil, β-aryophyllene, bisabolol, α-cedrene, carene delta, caryophylene acetate, caryophyllene, citronellol, cyclomethylene citronol, decyl acetate, ethyl butyrate, ethyllinalool, hexenol cis-3, kalsec pepper extract, lemon terpenes, lime terpenes, D-limonene (further referred to as limonene), mandarin terpenes, menthol, methyl salicylate, myrcene, nonyl acetate, octyl acetate, orange terpenes, α-pinene and β-pinene, tangerine terpene, α-terpeneol, α-terpinene, valencene, and combinations thereof. In an embodiment, the terpene is limonene, and/or D-limonene, optionally in combination with menthol.

In embodiments, the at least one terpene or related terpene can be administered in an amount sufficient to enhance the efficacy of the dicarboxylic acid ester in providing the effects described above. The one or more terpenes or terpenoids may be used in an amount within the range from, e.g., about 0.1% to about 20%, about 0.25% to about 10%, or from about 1% to about 5%, or from about 2% to about 4%, or any value or range therebetween.

### Methods

In embodiments, the disclosure provides a method for inducing an analgesic effect, thereby inhibiting pain in a subject, including administering to the subject a pharmaceutical composition including an azelaic acid ester, at least one solvent, and at least one terpene. In embodiments, the disclosure provides a method for inducing an analgesic effect, thereby inhibiting pain in a subject, includes administering to the subject the above-described pharmaceutical composition.

Inducing a localized analgesic effect may be accomplished by disrupting or increasing the fluidity of lipid rafts. Lipid rafts (also known as lipid microdomains) are discrete lipid domains present in the external leaflet of the plasma membrane. Lipid rafts are enriched in cholesterol, sphingomyelin, gangliosides, and sphingolipids such as monosialotetrahexosylganglioside1 (GM1). Lipid rafts influence membrane fluidity and membrane protein trafficking, thereby regulating neurotransmission and receptor trafficking. [Korade Z, et al., Neuropharmacology (2008) 55 (8): 1265-73].

In embodiments, the analgesic effect is induced due to the disruption of lipid rafts caused by contacting a dicarboxylic acid ester of Formula I with lipid rafts. When the dicarboxylic acid ester of Formula I is applied topically onto the subject (e.g., open wound, area around the wound, healthy skin, mucous membrane, oral mucosal wound), the dicarboxylic acid ester makes contacts with the lipid rafts located in the plasma membranes of the subject, disrupting the lipid rafts. In embodiments, the lipid raft contains GM1.

In embodiments, the lipid raft contains phospholipase D2 (PLD2). In embodiments, the dicarboxylic acid ester of Formula I disrupts lipid raft containing GM1. In embodiments, the disruption of the lipid raft by a dicarboxylic acid ester of Formula I caused PLD2 to depart the lipid raft, thereby activating TREK-1 and subsequently promoting the production of signaling lipid phosphatidic acid (PA).

Pain can be subdivided by type in a variety of ways. On the basis of time, pain can be classed as chronic or acute. Chronic pain is recognized as pain that lasts past normal healing time, usually for more than 3 months. Chronic pain affects about 20% of people worldwide and accounts for about 20% of physician visits. [Treede Z, et al., Pain (2015) 156(6):1003-7]. On the basis of mechanism, pain can be nociceptive, neuropathic or nociplastic. Nociceptive pain results from stimulation of pain receptors for issue injury (nociceptors) due to mechanical, chemical, or thermal stimuli. Neuropathic pain results from damage to components of the nervous system. Nociplastic pain arises from an alteration of nociceptive perception.

In embodiments, the subject is suffering from a pain. In embodiments, the pain is acute or chronic. In embodiments, the pain is nociceptive pain, neuropathic pain, or nociplastic pain.

In embodiments, the pain is acute. In embodiments, the subject can be in need of treatment for acute pain. Examples of acute pain include, but are not limited to, traumatic pain, procedural (surgery, dental, dermatologic, etc.) pain, wound pain, musculoskeletal pain (e.g., back pain, neck pain), toothache, infection (e.g., wound infection), and toxins (insect, animal, bacterial, fungal, etc.).

In embodiments, the pain is chronic. In some embodiments, the subject can be in need of treatment for chronic pain. Examples of chronic pain include, but are not limited to, fibromyalgia, arthritis pain, iliotibial band syndrome pain, tennis elbow pain, cancer pain, musculoskeletal pain (e.g., back pain, neck pain), temporomandibular joint disorder, trigeminal neuralgia, chronic headaches, pain associated with neurologic diseases (MS, diabetic neuropathy, etc.), neuroma, pelvic inflammatory disease, endometriosis, shingles and post-herpetic neuralgia, and infection (e.g., HIV) associated chronic neuropathy, chemotherapy-induced neuropathic pain, surgery-induced neuropathic pain, trauma-induced neuropathic pain, vulvodynia, atypical craniofacial pain, sciatica, phantom limb, odontalgia, and burning mouth syndrome.

In embodiments, the pain is nociceptive pain. In embodiments, the subject can be in need of treatment for nociceptive pain. Nociceptive pain is usually acute and develops in response to a specific situation. Examples of nociceptive pain include, but are not limited to, pain from sprains, burns, bruises, surgical procedures and bone fractures. Chronic nociceptive pain results from some conditions when pain extends beyond six months. Examples of chronic nociceptive pain include pain from cancer, rheumatoid arthritis, osteoarthritis, musculoskeletal conditions (e.g., back pain).

In embodiments, the pain is neuropathic pain. In embodiments, the subject can be in need of treatment for neuropathic pain. Neuropathic pain results from damage to components of the nervous system. Certain disease conditions can be the underlying cause of neuropathic pain. For example, a subject may be suffering from a metabolic disease *(e.g.,* diabetic neuropathy), an autoimmune disease *(e.g.,* multiple sclerosis), a viral infection (e.g., shingles and sequelae, postherpetic neuralgia), vascular disease *(e.g.,* stroke), trauma and/or cancer. [Campbell JN et al., Neuron (2006) 52(1):77-92; Dworkin RH et al., Arch Neurol (2003) 60; 1524-34]. In embodiments, the neuropathic pain is due to nerve damage arising from metabolic disease, trauma, ischemia or hemorrhage, inflammation, neurotoxicity, neurodegeneration, paraneoplastic, vitamin deficiency, or cancer. Examples of neuropathic pain include, but are not limited to, post herpetic (or post-shingles) neuralgia, reflex sympathetic dystrophy / causalgia (nerve trauma), components of cancer pain, phantom limb pain, entrapment neuropathy (e.g., carpal tunnel syndrome), and peripheral neuropathy (widespread nerve damage).

In embodiments, the pain is nociplastic pain. In embodiments, the subject can be in need of treatment for nociplastic pain. Nociplastic pain is pain that arises from altered nociception despite no clear evidence of actual or threatened tissue damage causing the activation of peripheral nociceptors or evidence for disease or lesion of the somatosensory system causing the pain. [Chimenti RL et al., Phys Ther. (2018) 98(5): 302-314].

In embodiments, the pain is selected from acute pain, chronic pain, nociceptive pain, neuropathic pain, nociplastic pain, traumatic pain, chemical pain, burn pain, ischemic pain, insect bite pain, prickling pain, musculoskeletal pain (e.g., back pain, neck pain), rheumatoid arthritis pain, post-surgical pain, bone pain (e.g., osteoarthritis), pain due to various skin conditions (e.g., acne, psoriasis, hidradenitis suppurativa, eczema, rosacea).

In embodiments, the reduction of pain (or the reduction of a pain response) is due to the modulation of PRR activity. In embodiments, the reduction of a pain response (or the reduction of a pain response) is due to the modulation of NOD receptor activity. In embodiments, the reduction of a pain response (or the reduction of a pain response) is due to the modulation of Dectin receptor activity.

### Synthesis of dicarboxylic acid esters

The dicarboxylic acid ester of Formula I may be obtained commercially or prepared by various methods known in the art. In embodiments, the dicarboxylic acid ester can be prepared via direct formation of the ester from the requisite acid and an alcohol. This condensation may be achieved by the dehydration of the reaction mixture with a suitable agent or by heating a mixture of the acid and alcohol. In embodiments, the dicarboxylic acid ester can be prepared by reacting an alcohol with an activated form of the acid. Activated forms of the acid include acid halides, acid anhydrides including both homo and hetero anhydrides, the reaction of the internal anhydride of the parent acid with the requisite alcohol, esters and anhydrides of both the acid and the alcohol which are formed by reaction of the requisite acid or alcohol with p-toluene sulfonyl chloride to produce the tosyl anhydride or ester which is subsequently reacted with the alcohol or acid respectively to produce the desired final ester. Similarly, one could substitute a simple organic acid anhydride, such as acetic acid anhydride, for the p-toluene sulfonyl chloride. In addition, one could start with one ester selected from among the desired compositions of matter and by the means of solution of the ester in a desired alcohol in the presence of an appropriate acidic or basic catalyst effect a conversion of the starting ester of the acid to an ester wherein the alcohol becomes that in which the reaction is carried out which method is also known to the art as transesterification.

For example, one could start with the dimethyl ester of the acid and by solution of the ester in ethanol in the presence of an acid or base one could cause the facile formation of the diethyl ester of the acid. In addition, if a mixed ester of the acid were desired, one could utilize an appropriately composed solution of the two or more desired alcohols in any of the methods herein described.

One could resort to the use of halogenated intermediates or ingredients to form the required esters. For example, thionyl chloride will chlorinate both acids and alcohols, thereby resulting in the acyl and alkyl chlorides. These acyl and alkyl chlorides may then be further reacted with the desired alcohol or acid respectively to produce the desired ester products. Other common halogenating agents include for example oxalyl chloride and the chlorides and bromides of phosphorous such as phosphorous penta or trichloride and penta or tribromide or phosphorous oxychloride.

It is commonly practiced to form esters through the action of a strong base on a mixture of the acid and the alcohol. Examples of strong bases include lithium aluminum hydride and other metal hydrides, alkali metal alkoxides such as sodium ethoxide and diisobutyl aluminum hydride and so on.

### Methods of Administration

The pharmaceutical composition of the present disclosure may be administered to a subject in a variety of ways. For example, the pharmaceutical compositions can be administered topically, transdermally, intravenously, subcutaneously, intramuscularly or orally. The pharmaceutical composition may be applied locally at and/or around the target area or the area where treatment is desired.

The treatment regime can vary depending upon various factors typically considered by one of ordinary skill in the art. These factors include the route of administration, the nature of the formulation, the nature of the patient's illness, the subject's size, weight, surface area, age, gender, other drugs being administered to the patient, and the judgment of the attending physician. The pharmaceutical compositions can be administered along with or in addition to other treatments for an inflammatory condition or pain reduction.

The pharmaceutical compositions can be administered in combination with one or more additional therapeutic agents for the treatment of pain and/or inflammation. The one or more additional therapeutic agents may be administered by the same or different routes of administration. The one or more additional therapeutic agents may include pain relievers, anti-inflammatory agents, anesthetic agents, antibiotics, and antifungals.

In embodiment, the pharmaceutical composition is administered to a subject topically on the skin of the subject, for example, in areas located at or at least within the vicinity of an area where treatment is desired. In embodiment, the pharmaceutical composition is administered to a subject by rubbing it topically against the skin, which allows the composition (or at least, the dicarboxylic acid ester) to be absorbed by the skin.

In embodiments, for prophylactic treatments, the pharmaceutical compositions can be administered to a subject at a time period of from 96 hours to immediately before, from 72 hours to immediately before, from 48 hours to immediately before, from 24 hours to immediately before, from 12 hours to immediately before, from 8 hours to immediately before, from 4 hours to immediately before, from 2 hours to immediately before, from 1 hour to immediately before, or from 0.5 hour to immediately before a procedure (or surgery), or any range derivable therein immediately before the procedure. The pharmaceutical compositions may be applied topically to an area of healthy skin, at any time, for example, in one embodiment, before hiking.

In embodiment, for purpose of prophylactic treatments, the pharmaceutical composition is administered to a subject locally or topically prior to a procedure, such as, a venipuncture, an injection, incision, hair removal, tattoo application and removal.

In embodiment, the pharmaceutical composition is administered to a subject locally or topically during or after a procedure, such as, a venipuncture, an injection, incision, hair removal, tattoo application and removal.

In embodiment, the pharmaceutical composition is applied once, or more than once to a subject. For example, the pharmaceutical composition may be administered at predetermined intervals. In embodiments, for instance, the pharmaceutical composition may be applied once per day, twice per day, 3 times per day, 4 times per day, or more than 4 times per day, or once every other day, once every three days, once every four days, etc.

In embodiment, the pharmaceutical composition is administered to the subject in a therapeutically effective dose. When administered to a subject, therapeutically effective amounts will depend on the particular condition being treated and the desired outcome.

The pharmaceutical compositions can be administered to a subject in need at about every 1 to about 24 hours, about every 1 to about 12 hours, about every 2 to about 8 hours, about every 2 to about 6 hours, about every 4 to about 6 hours, about every 4 to about 8 hours, about every 12 hours, about every 24 hours, about every 48 hours, or more often. In embodiments, the pharmaceutical composition can be administered once, twice, three times, four times, five times, six times, seven times, eight times, or more often daily, or any combination thereof. In embodiments, the pharmaceutical composition can also be administered daily, every other day, every two days, every three days, every four days, or less often, or any combination thereof. In embodiments, the pharmaceutical composition can be administered to a subject in need for a duration of from 1 day to 30 days, from 1 day to 25 days, from 1 day to 20 days, from 1 day to 15 days, or from 1 day to 10 days.

### Formulations

In embodiments, the pharmaceutical compositions of the disclosure can be formulated for delivery via any route of administration known in the art, including but not limited to topical, transdermal, intravenous, subcutaneous, intramuscular, or oral administration.

Proper formulation is dependent upon the route of administration chosen. Any of the well-known techniques, carriers, and excipients as understood in the art may be used e.g., those disclosed in Remington's Pharmaceutical Sciences, 18th ed, (Mack Publishing Company: Easton, PA., 1990) incorporated herein by reference. The pharmaceutical compositions disclosed herein may be manufactured in any manner known in the art, such as by means of conventional mixing, dissolving, granulating, levigating, emulsifying, encapsulating, entrapping or compression processes.

The pharmaceutical compositions include those suitable for topical, transdermal, intravenous, subcutaneous, intramuscular, or oral administration, although the most suitable route may depend upon, for example, the condition and disorder of the recipient.

In embodiments, the pharmaceutical composition is suitable for topical and transdermal administrations. Topical and transdermal administration of azelaic acid esters of the present disclosure can be in the form of a processed gel, cream, lotion, solution, ointment, suspension, or emulsion. These pharmaceutical compositions may further include one or more suitable excipient disclosed herein.

Parenteral administration, such as intravenous, subcutaneous, intramuscular administration of a dicarboxylic acid ester of Formula I can be in the form of solutions, suspensions, or emulsions. In one embodiment, these formulations are prepared in a saline solution. These pharmaceutical compositions may further include one or more suitable excipient disclosed herein.

In embodiments, the pharmaceutical composition is suitable for oral administration. The pharmaceutical compositions may conveniently be presented in unit dosage forms and may be prepared by any of the methods well known in the art of pharmacy. Typically, these methods include the step of mixing a dicarboxylic acid ester of Formula I, and optionally any co-administered active ingredient, with the carrier which constitutes one or more accessory ingredients. In general, the pharmaceutical compositions are prepared by uniformly and intimately mixing the active ingredients with liquid carriers or finely divided solid carriers or both and then, as necessary, shaping the product into the desired composition. The pharmaceutical composition and any optional secondary active ingredient, suitable for oral administration may be presented as discrete units such as capsules, cachets or tablets each containing a predetermined amount of the active ingredient(s); as a powder or granules; as a solution or a suspension in an aqueous liquid or a non-aqueous liquid; or as an oil-in-water liquid emulsion or a water-in-oil liquid emulsion. The active ingredient(s) may also be presented as a bolus, electuary or paste. These pharmaceutical compositions may further include one or more suitable excipient disclosed herein.

### Excipients

In embodiments, the pharmaceutical composition of the disclosure may further include one or more excipients. The excipient may include a carrier, for example, water-insoluble polysaccharide or oligosaccharide. Examples of carriers include, but are not limited to, cellulose acetate, cellulose acetate butyrate, cellulose acetate propionate, cellulose acetate phthalate, chitosan, β-cyclodextrin, ethyl cellulose, hydroxypropylmethyl cellulose phthalate (HPMCP), microcrystalline cellulose, starch, and any combination thereof.

The excipient may include a thickening agent, for example, a water-soluble polysaccharide. Examples of thickening agents include, but are not limited to, hydroxy propyl methyl cellulose (HPMC), acacia, alginic acid, colloidal silicone dioxide, carboxymethylcellulose calcium, gelatin, hydroxy propyl cellulose, hydroxyl propyl cellulose (hypromellose), methyl cellulose, sucrose, sodium alginate, sodium carboxy methyl cellulose, and any combination thereof.

In embodiments, the pharmaceutical composition of the disclosure may further include one or more pharmaceutical excipients, for example ascorbic acid, EDTA dihydrate, glycerin, citric acid monohydrate, sodium citrate dihydrate, sodium benzoate, sodium propionate, 70% sorbitol solution, sucralose, FD&C Yellow #6, artificial flavor (e.g., artificial peppermint flavor, artificial fruit flavor), purified water, or any combination thereof.

In embodiments, the pharmaceutical composition of the disclosure may include a preservative, Suitable preservatives include, but are not limited to, mercury-containing substances such as phenylmercuric salts (e.g., phenylmercuric acetate, borate and nitrate) and thimerosal; stabilized chlorine dioxide; quaternary ammonium compounds such as benzalkonium chloride, cetyltrimethylammonium bromide and cetylpyridinium chloride; imidazolidinyl urea; parabens such as methylparaben, ethylparaben, propylparaben and butylparaben, and salts thereof; phenoxyethanol; chlorophenoxyethanol; phenoxypropanol; chlorobutanol; chlorocresol; phenylethyl alcohol; disodium EDTA; benzoic acid, benzyl alcohol and sorbic acid and salts thereof.

In embodiments, the pharmaceutical composition of the disclosure may include one or more acceptable pH adjusting agents and/or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in a pharmaceutically acceptable range. In embodiments, the pH of the pharmaceutical composition of the disclosure may be from pH 4 to pH 7.5.

In embodiments, the pharmaceutical composition of the disclosure may include a sugar alcohol. Examples of sugar alcohols include, but are not limited to, mannitol, glycerol, galactitol, fucitol, inositol, volemitol, maltotriitol, maltoetetraitol, polyglycitol, erythritol, threitol, ribitol, arabitol, xylitol, allitol, dulcitol, glucitol, sorbitol, altritol, iditol, maltitol, lactitol, isomalt, and any combination thereof.

In embodiments, the pharmaceutical composition of the disclosure may include an additive. Examples of additives include, but not limited to, diluents, binders, surfactants, lubricants, glidants, coating materials, plasticizers, coloring agents, flavoring agents, or pharmaceutically inert materials. Examples of diluents include, for example, cellulose; cellulose derivatives such as microcrystalline cellulose and the like; starch; starch derivatives such as corn starch, cyclodextrin and the like; sugar; sugar alcohol such as lactose, D-mannitol and the like; inorganic diluents such as dried aluminum hydroxide gel, precipitated calcium carbonate, magnesium aluminometasilicate, dibasic calcium phosphate and the like. Examples of binders include, for example, hydroxypropylcellulose, methylcellulose, hydroxypropylmethylcellulose (hydroxypropyl methylcellulose), povidone, dextrin, pullulane, hydroxypropyl starch, polyvinyl alcohol, scacia, agar, gelatin, tragacanth, macrogol and the like. Examples of surfactants include, for example, sucrose esters of fatty acids, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate, lauromacrogol, quaternary ammonium salts (e.g., benzyldimethyltetradecylammonium chloride hydrate, benzethonium chloride, benzylcetyldimethylammonium chloride hydrate, benzyldimethylstearylammonium chloride hydrate, benzyldodecyldimethylammonium chloride dihydrate, benzyldodecyldimethylammonium bromide), and the like. Examples of lubricants include, for example, stearic acid, calcium stearate, magnesium stearate, talc and the like. Examples of glidants include, for example, dried aluminum hydroxide gel, magnesium silicate and the like. Examples of coating materials include, for example, hydroxypropylmethyl cellulose 2910, aminoalkyl methacrylate copolymer E, polyvinylacetal diethylaminoacetate, macrogol 6000, titanium oxide and the like. Examples of plasticizers include, for example, triethyl citrate, triacetin, macrogol 6000 and the like.

### Kits

In embodiments, the disclosure also provides a kit that includes a pharmaceutical composition of the present disclosure, especially for topical administration, a wipe to clean the site, a swab or brush to spread the applied material, an adhesive dressing to cover the site; optionally, the kit may include a disposal bag or container; optionally, the kit may include an absorbent element; optionally, the kit may include a pair of gloves (e.g., sterile nitrile or latex gloves).

### EXAMPLES

### MATERIALS AND METHODS

Chemicals: Diesters of azelaic acid were synthesized from azelaic acid and ethyl alcohol using acid-catalyzed esterification followed by fractional distillation to 99% purity as determined by gas chromatography-mass spectrometry (GC-MS) as described in Izbicka E, Streeper R and Louden C: Adaptive membrane fluidity modulation: A feedback regulated homeostatic system and target for pharmacological intervention In Vivo 35(3073-3095, 2021. Unless stated otherwise, chemicals were sourced from Sigma-Aldrich (St. Louis, MO) and Thermo Fisher Scientific (Waltham, MA). Terpenes were sourced from Vigon International (East Stroudsburg, PA).

Touch Test Sensory Evaluator kit (North Coast Medical Inc, Morgan Hill, CA) includes 20 monofilaments that exert target forces between 0.25 and 512 millinewtons (mN), corresponding to 0.008 and 300 g and coded numerically with the logarithm of the applied force in milligrams as follows: 1.65, 2.36, 2.44, 2.83 (normal sensation), 3.22, 3.61, 3.84 (diminished light touch), 4.08, 4.17, 4.31 (diminished protective sensation) and 4.56, 4.74, 4.93, 5.07, 5.18, 5.46, 5.88, 6.10, 6.45, 6.65 (loss of protective sensation). Notably, logarithmic transformation makes the variance (scatter) across the different treatment groups more uniform. See Mills C, et al., "Estimating efficacy and drug ed50's using von frey thresholds: Impact of weber's law and log transformation," J Pain 13(6): 519-523, 2012

Cutaneous mechanical sensitivity assay (CMS) using the monfilaments to test sensory levels and obtain data on the status of diminishing or return in sensibility, followed the modified protocols of Rolke R et al., "Quantitative sensory testing: A comprehensive protocol for clinical trials," Eur J Pain 10(1): 77-88, 2006, and Kostek M, et al., "A protocol of manual tests to measure sensation and pain in humans," J Vis Exp 118), 2016. The tests were conducted at approximately the same time in the morning. A subject was positioned face down and prone on a flat surface and a circular testing area with a radius of 10 cm was marked on the back of the left and right popliteal area behind the knees. To establish a baseline of minimum force to exert a touch sensation, the areas were randomly touched with a monofilament in a series of ten measurements (5 per each side) using monofilaments of increasing force. Each touch was recorded and the subject reported a touch sensation if experienced. A positive score for a monofilament as was defined as ≥6 reported incidents of the touch sensation.

Solutions of test articles were prepared in isopropyl alcohol. To test DEA and DMSO at their highest equimolar concentrations of approximately 3M each, stock solutions were prepared neat without the diluent and adjusted for the densities of the two compounds, corresponding to 78% v/v DEA and 22% v/v DMSO. These compounds were evaluated as single reagents and in variable ratios of equimolar stocks of DEA at 78, 58, 39 and 19% v/v, and DMSO at 22, 16, 11, and 5% v/v (rounded to the nearest integer). With additional components, respective highest concentrations of DEA and DMSO were 77% and 21%.

Following baseline evaluations, test article solutions were applied in a volume of 0.5 ml per testing area and gently rubbed in for 15 seconds (Day 1, time = 0). Mechanical sensitivity tests were then performed at the earliest time of 5 minutes (0.1 h) and then at 1, 4, 8, 24, 48, 72, and 96 hours. The assay was completed when the touch sensation returned to the baseline and further referred to as the time to the baseline (TBL). Unless specified otherwise, the assays were performed with10 replicate measurements.

The area under the curve (AUC) for the course of each test from t = 0 until return to baseline sensitivity was determined using SigmaPlot version 14.5 (Inpixion, Palo Alto, CA). Statistical analysis was done using Students t-test; p values <0.05 were considered significant and <0.005; highly significant.

The hemolysis assay in peripheral human blood using bee venom preparation with the active hemolytic component PLA2 was performed in triplicate repeats as described in Streeper RT and Izbicka E , "Diethyl azelate for the treatment of brown recluse spider bite, a neglected orphan indication," In Vivo 36(1): 86-93, 2022. Briefly, a stock solution of bee venom was diluted in phosphate buffered saline (PBS) with 0.5% Tween (diluent) to yield the extent of hemolysis comparable Triton-X used as a positive control. All test articles were also prepared in the same diluent. The reagents were preincubated at room temperature for 5 minutes and a 30-minute reaction was initiated by the addition of the erythrocyte suspension.

To evaluate the blood glucose effects of DEA alone and the mixture of DEA, DMSO and limonene (77%, 21%, and 2% respectively), the solutions were taken orally in the volumes of 12.5 µl (-12.5 mg). Blood glucose levels (measured in 8 repeats of each treatment) were quantified prior to the dosing at time 0 and then every 15 min over 2 hours using OneTouch Ultra2 glucometer (LifeScan, Malvern, PA) and UniStrip1 test strips (UniStrip Technologies, Charlotte, NC) as described by the manufacturers.

### Example 1 - Evaluation of DEA and DMSO in cutaneous mechanical sensitivity assays

This experiment demonstrates that DEA and DMSO used in combination exhibit unexpected synergistic activity and non-linear concentration dependence in cutaneous mechanical sensitivity assay (CMS).

The CMS assays (n=20) established that the baseline of sensitivity without any treatment was with the monofilament number 2.36; having a bending force of 0.02 g. DEA and DMSO were tested as single agents or as mixtures of DEA and DMSO. The results summarized in **Table 1** (wherein the relative molar ratio of 1 corresponds to 78% DEA and 22% DMSO) indicate that increased molar ratios apparently did not correlate with increased time to baseline (TBL). The unusual relationship can be appreciated by comparing the data for DEA and DMSO, especially at the molar ratios above 0.75. Table 1 below shows the relationship between DEA and DMSO relative molarity (Rm) and the time to baseline in cutaneous mechanical sensitivity assay.

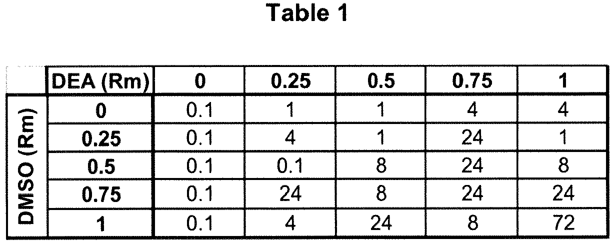

**Table 2** presents more extensive data in the matrix of DEA and DMSO concentrations and the endpoints expressed as time to baseline (TBL) for the mixture of the two compounds and with a correction (TBL-C) for the contribution of DMSO to the mixture when the effect of DEA alone has been subtracted from TBL of the mixture. The TBL values were uniformly reproducible thus no standard deviations are shown. Both AUC values and the standard deviations are presented. The maximum highly significant effect was observed at TBL of 72 h (TBL-C of 68) with the percentage ratios of DEA and DMSO of 78/22 and 58/22. The latter tests utilized fibers 4.74, 4.03 and 5.07, which applied a bending force of 6-10 g. The results demonstrate a significant loss of protective sensation in comparison with a control with no treatment. **Table 2** below shows the relationship between DEA and DMSO concentrations, AUC and the time to baseline in cutaneous mechanical sensitivity assay.

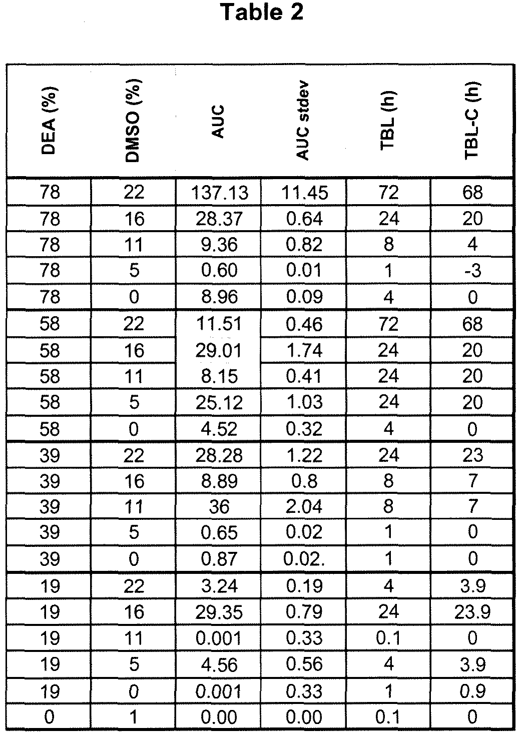

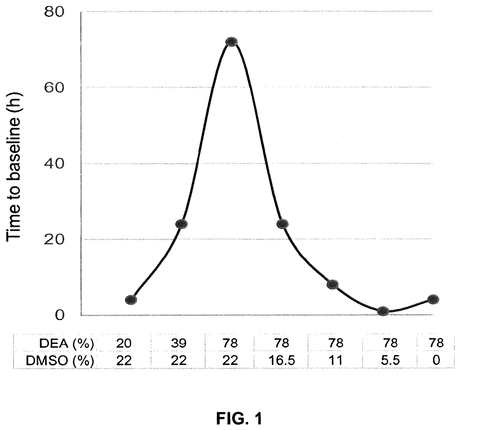

**Fig. 1** highlights unexpected properties of DMSO in combinations with DEA. DMSO under 10% was ineffective but the analgesic effect steeply increased to a maximum at 22% DMSO and 78% DEA, and then sharply declined at lower concentrations of DEA. These data show that the enhancement of the analgesic effect of DEA is NOT due merely to enhancement of tissue penetration by DMSO.

### Example 2 - Screen of topical analgesics, turpentine and terpenes

These experiments demonstrated unexpected superior activity of turpentine, limonene, pinene and menthol in certain combinations with DEA and DMSO.

Initial CMS assays performed with select topical analgesics. The time to baseline (TBL) values for 4% Lidocaine and Voltaren (1.16% diclofenac) were 4 h and 8 h, respectively. Since turpentine (also known as terpentine) is a component of some analgesics, it was also examined in the CMS screen. A mixture of 1% and 2% turpentine with 21% DEA and 77% DMSO yielded TBL values ranging from 24 h to 48 h relative to the vehicle control.

Turpentine is a highly variable mixture of constituents. The product made in the United States typically contains α-pinene (75 to 85%). varying amounts of β-pinene (up to 3%), camphene (4 to 15%), limonene (5 to 15%), and low percentages of 3-carene, and terpinolene.

To evaluate relative contributions of turpentine components in the CMS tests, screens utilized a mixture of 19% DEA and 5% DMSO plus the following turpentine components and related terpenes, with all compounds tested individually at 2% v/v: amyl alcohol, arnica extract, arnica oil, β-aryophyllene, bisabolol, α-cedrene, carene delta, caryophylene acetate, caryophyllene, citronellol, cyclomethylene citronol, decyl acetate, ethyl butyrate, ethyllinalool, hexenol cis-3, kalsec pepper extract, lemon terpenes, lime terpenes, D-limonene (further referred to as limonene), mandarin terpenes, menthol, methyl salicylate, myrcene, nonyl acetate, octyl acetate, orange terpenes, α-pinene and β-pinene, tangerine terpene, α-terpeneol, α-terpinene, and valencene. In addition, cannabinoid oil (30%) and a synthetic capsaicinoid pelargonic acid vanillylamide (PAVA) (0.25% to 4%) was examined. In all cases, TBL was at least 1 h for the examined compounds but the differences between the treatments and control were not statistically significant except for menthol, α-pinene, and D-limonene. These compounds were further evaluated in **Example 3** below.

### Example 3 - Evaluation of multi-component mixtures in CMS assays

This example illustrates unexpected enhancement of TBL by certain combinations of DEA, DMSO, menthol, α-pinene, and limonene. Further, among the diesters of azelaic, suberic and sebacic acid in the absence or presence of limonene, only DEA ± limonene significantly suppressed CMS.

**Table 3** summarizes the effects of DEA and DMSO at variable concentrations in combination with limonene. The last column shows the values TBL corrected for the contribution of the DEA and DMSO without limonene (TBL-C). Bold type in TBL-C show the values above the contribution of DEA and DMSO. **Table 3** below shows the relationship between DEA, DMSO and limonene concentrations, AUC and the time to baseline in cutaneous mechanical sensitivity assay.

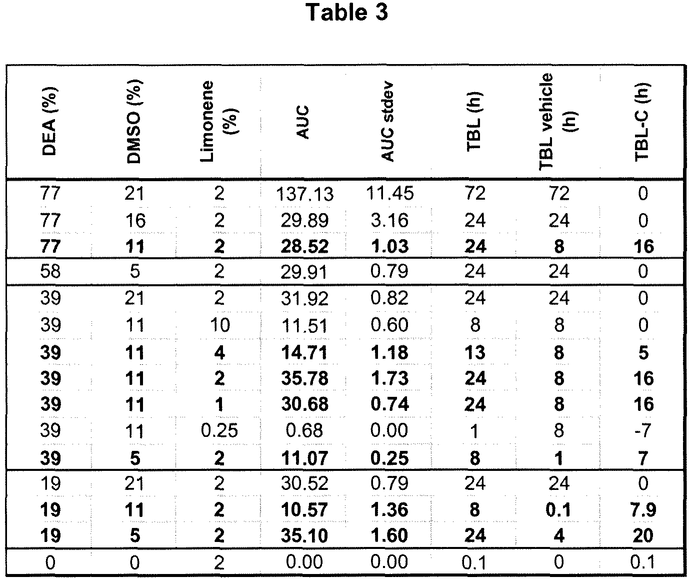

As shown in **Table 3,** when 2% limonene was used, the longest TBL-C of 20 h was observed with equimolar DEA and DMSO (19% and 5%, respectively). The values of TBL-C decreased to 16 h with equimolar DEA and DMSO (39% and 11%, respectively). However, 2% limonene did not further enhance the combined activity of equimolar DEA and DMSO that was observed at their highest respective concentrations of 77% and 21%.

It is noteworthy that 2% limonene synergized with DEA and DMSO at lower percentages thereof. As shown in Tables 2 and 3, 39% DEA and 11% DMSO mixture had a TBL-C of 7 h without limonene but TBL-C of 16 h in the presence of 1% or 2% limonene.

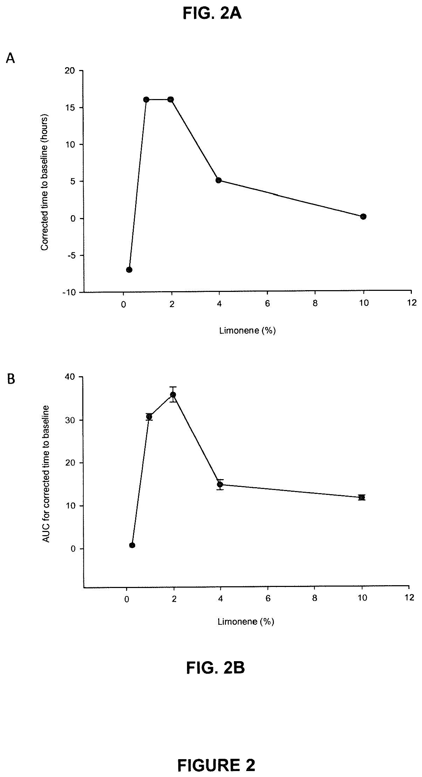

A dose response of limonene was evaluated with 20% DEA and 7% DMSO **(****Fig. 2A and Fig. 2B****).** The highest value of TBL of 16 h, corrected for the contribution of DEA and DMSO, was observed with 4% and 2% limonene. Ten percent limonene had no effect, while 0.25% limonene decreased the TBL to below the DEA plus DMSO level. Limonene also exhibited a bell-curve dose response.

Given significant enhancement of the TBL by 2% limonene in DEA/DMSO mixtures, other medium chain fatty acid diesters were also examined at 39% concentrations in 11% DMSO ± 2% limonene. As shown in **Fig. 3A** and **Fig. 3B****,** DEA was superior to other diesters and limonene added significantly to the effect of DEA. Likewise, limonene demonstrated a peak analgesic activity at between 1 and 2% but the contribution of limonene decreased at higher concentrations with no added benefit above 4% **(****Fig. 2A** and **Fig. 2B****).**

In a head-to-head comparison of pinene isomers **(Table 4),** 2% α-pinene was vastly superior to β-pinene in the mixtures of DEA/DMSO of 39%/11%, and 19%/5%. The activity of 2% α-pinene was comparable to that of 2% limonene at the same equimolar DEA/DMSO concentrations (see Table 2). However, the effect of mixture of 2% limonene and 2% α-pinene in DEA/DMSO was unremarkable. **Table 4** below shows the relationship between DEA, DMSO, limonene and pinene concentrations, AUC and the time to baseline in CMS assay.

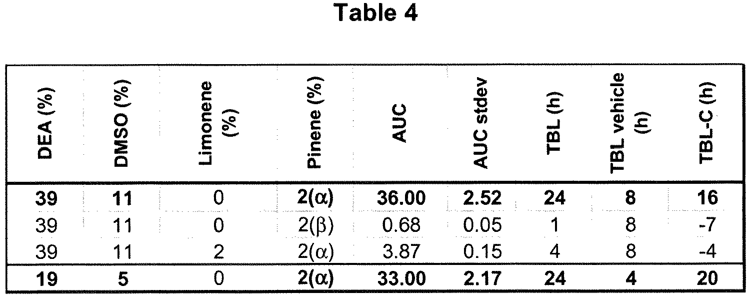

As shown in **Table 5,** menthol (0.1% to 4%) combined with 39% DEA was ineffective. A surprising augmentation of analgesic effect was realized by adding 0.1% menthol to DEA/DMSO mixtures at the percentage ratios of 39/11 and 19/5. The analgesic effect of DEA/DMSO/limonene at ratios of 39/11/2 and 19/5/2 was even more significantly enhanced by 0.1% menthol. The maximum observed TBL of 48 h for DEA/DMSO/limonene/menthol (39/11/2/0.1) was significantly greater when compared with the DEA/DMSO/limonene mixture without menthol (TBL-C of 16 h; see **Table 3). Table 5** below shows the effects of menthol on the activity of the mixture of DEA, DMSO and limonene on the duration of sensitivity suppression in cutaneous mechanical sensitivity assays.

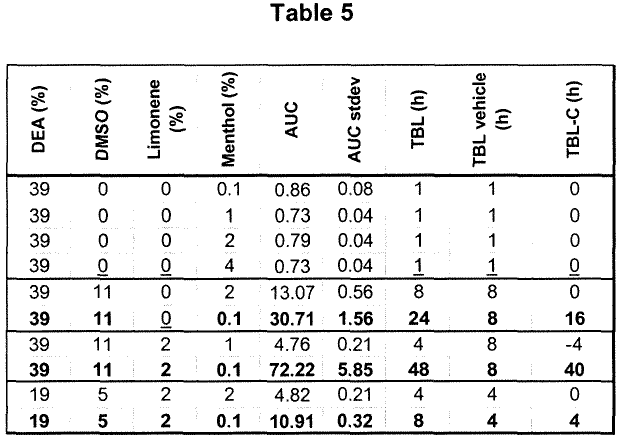

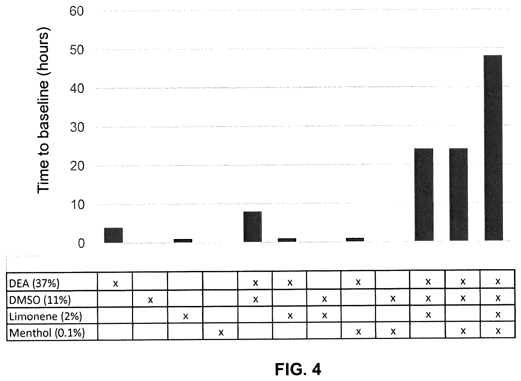

**Fig. 4** summarizes the effects of 39% DEA, 11% DMSO, 2% limonene and 0.1% menthol as single agents and in combinations thereof on the duration of sensitivity suppression in CMS assays. Both the 4-component and 3-component mixtures showed superior activity to any single reagents or 2-component mixtures.

### Example 4 - Comparison between oral DEA and a mixture of DEA, DMSO and limonene on blood glucose levels

This experiment provides a comparison between oral DEA and mixtures of DEA, DMSO, and limonene on blood glucose levels.

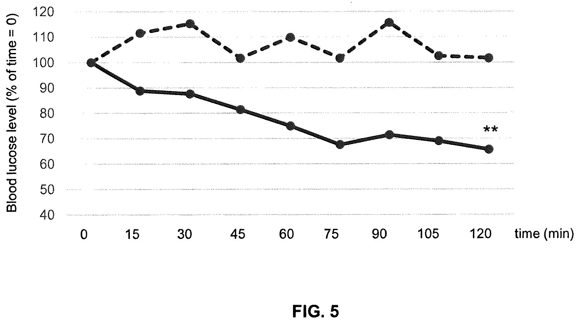

**Fig. 5** shows blood glucose levels after a single oral dose of 12.5 ul DEA corresponding to 0.17 mg/kg of DEA (broken top line) and a 12.5 ul of a mixture of 77% DEA, 21% DMSO and 2% limonene corresponding to 0.13 mg/kg of DEA (solid bottom line). The average difference of 39% between the two formulas on average glucose levels at 2 h is highly significant (p= 0.001).

In the past, oral administration of DEA was shown to exert optimum blood glucose suppression at 1 mg/kg. Current results suggest that the mixture 77% DEA, 21% DMSO and 2% limonene is approximately 5.8 times more potent than DEA alone. An embodiment therefore includes a composition containing from about 70 to about 80% DEA, from about 19 to about 22% DMSO, and from about 1 to about 3% limonene, or alternatively from 76-78% DEA, from 20.5 to 21.5% DMSO, and from 0.5 to 1.5% limonene.

### Example 5 - Evaluation of mixture composition of hemolysis induced by PLA2

This example shows an unusual bell-shaped dose and concentration effect of a mixture of DEA, DMSO and limonene compared to single reagents on the extent of hemolysis induced by PLA2 in bee venom.

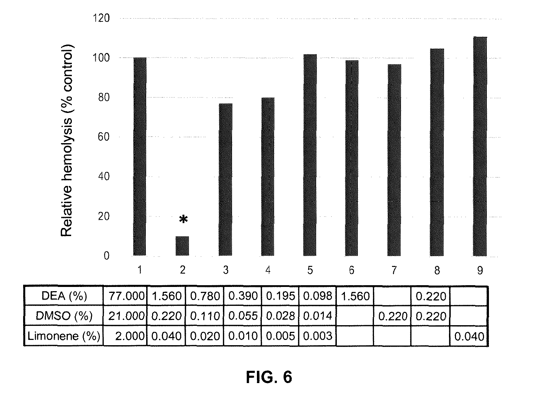

In **Fig. 6****,** bars 1-5 show a bell-shaped hemolysis inhibitory activity of mixtures of DEA, DMSO, and limonene. Notably, the maximum inhibition was measured at 50-fold lower concentrations than those effective in CMS. At the concentrations corresponding to the maximum inhibition, individual components were ineffective in this assay (bars 6-9). In addition, a comparison of three diethyl esters (C-8; suberate, C-9; azelate, and C-10; sebacate; all at 5%) demonstrated that diethyl sebacate significantly suppressed hemolysis by 60%.

While the embodiments have been described with reference to particularly preferred features, examples, and preferred embodiments, those skilled in the art will appreciate that various modifications may be made to these preferred embodiments without departing from the spirit and scope of the invention.

## Claims

1. A pharmaceutical composition comprising:
a dicarboxylic acid ester of Formula I:
R₂OOC-(CH₂)ₙ-COOR₁
wherein n is between 4 and 10, and each R₁ and R₂ are independently a C1 to C6 saturated straight or branched alkyl group;
at least one solvent; and
at least one terpene.

2. The pharmaceutical composition as claimed in claim 1, wherein the dicarboxylic acid ester of Formula I is an azelaic acid ester selected from diethyl azelate, diisopropyl azelate, and mixtures thereof.

3. The pharmaceutical composition as claimed in claim 2, wherein the azelaic acid ester is diethyl azelate.

4. The pharmaceutical composition as claimed in claim 1, wherein the dicarboxylic acid ester of Formula I is present in an amount with the range of from about 30% to about 90%, by weight of the composition.

5. The pharmaceutical composition as claimed in claim 1, wherein the at least one solvent is selected from the group consisting of dimethyl formamide, dimethyl sulfoxide (DMSO), dihydrolevoglucosenone (CYRENE^{™}), N-methylpyrollidone (NMP), halothane, isoflurane, enflurane, deslufrane, and sevoflurane and mixtures and combinations thereof.

6. The pharmaceutical composition as claimed in claim 5, wherein the solvent is DMSO.

7. The pharmaceutical composition as claimed in claim 1, wherein the at least one solvent is present in an amount with the range of from about 10% to about 50%, by weight of the composition.

8. The pharmaceutical composition as claimed in claim 1, wherein the at least one terpene is a terpene or terpenoid selected from the group consisting of arnica extract, arnica oil, β-aryophyllene, bisabolol, α-cedrene, carene delta, caryophylene acetate, caryophyllene, citronellol, cyclomethylene citronol, decyl acetate, ethyl butyrate, ethyllinalool, hexenol cis-3, kalsec pepper extract, lemon terpenes, lime terpenes, D-limonene (further referred to as limonene), mandarin terpenes, menthol, methyl salicylate, myrcene, nonyl acetate, octyl acetate, orange terpenes, α-pinene and β-pinene, tangerine terpene, α-terpeneol, α-terpinene, valencene, and mixtures and combinations thereof.

9. The pharmaceutical composition as claimed in claim 8, wherein the terpene is D-limonene.

10. The pharmaceutical composition as claimed in claim 1, wherein the at least one terpene is present in an amount with the range of from about 1% to about 5%, by weight of the composition.

11. A pharmaceutical composition comprising:
from about 70% to about 80% diethyl azelate;
from about 19 to about 22% DMSO; and
from about 1% to about 3% limonene.

12. A method of inducing an analgesic effect and inhibiting pain in subject comprising administering to the subject a therapeutically effective amount of a composition comprising:
a dicarboxylic acid ester of Formula I:
R₂OOC-(CH₂)ₙ-COOR₁
wherein n is between 4 and 10, and each R₁ and R₂ are independently a C1 to C6 saturated straight or branched alkyl group;
at least one solvent; and
at least one terpene.

13. The method as claimed in claim 12, wherein the dicarboxylic acid ester of Formula I is diethyl azelate.

14. The method as claimed in claim 12, wherein the dicarboxylic acid ester of Formula I is present in an amount with the range of from about 30% to about 90%, by weight of the composition.

15. The method as claimed in claim 12, wherein the at least one solvent is DMSO.

16. The method as claimed in claim 12, wherein the at least one solvent is present in an amount with the range of from about 10% to about 50%, by weight of the composition.

17. The method as claimed in claim 12, wherein the at least one terpene is limonene.

18. The method as claimed in claim 12, wherein the at least one terpene is present in an amount with the range of from about 1% to about 5%, by weight of the composition.

19. A method of inducing an analgesic effect and inhibiting pain in subject comprising administering to the subject a therapeutically effective amount of a composition comprising:
from about 70% to about 80% diethyl azelate;
from about 19 to about 22% DMSO; and
from about 1% to about 3% limonene.
